# EUROPEAN PATENT APPLICATION

(11) **EP 2 856 951 A1**
(43) Date of publication of application: **08.04.2015**
(21) Application number: 13187251.7
(22) Date of filing: 03.10.2013
(51) Int. Cl.: A61B 17/17

(54) **Surgical guide system**

(71) Applicant: Materialise N.V., 3001 Leuven (BE)
(72) Inventor: Faber, Nils, 3000 Leuven (BE); Laes, Philippe, 3000 Leuven (BE)
(74) Representative: EIP

(57) **Abstract**

Provided herein are guide systems for use in surgical interventions. More particularly, the guide system allows the checking of a surgical procedure according to a predetermined surgical plan in a limited surgical window.

## Description

### FIELD OF THE INVENTION

Provided herein are guide systems for use in surgical interventions. More particularly, the guide system allows the checking of a surgical procedure according to a predetermined surgical plan in a limited surgical window.

### BACKGROUND OF THE INVENTION

For a number of years, the combination of virtual pre-operative planning and patient-specific surgical instruments has been available for performing complex surgical interventions. This combination allows the surgeon to meticulously plan the surgery in advance on a virtual 3D model, and then execute this planning in the operating room by using patient-specific surgical instruments, which are unique to the patient and the planned surgery. The patient-specific instruments typically fit on a part of the patient anatomy and provide guidance to the surgeon.

However, even in complex surgical interventions where the surgeon follows precisely the preoperative plan, a number of manipulations and steps are to be performed by the surgeon relying mainly on the experience and assessment of the surgeon during the surgical procedure. This is for instance true for surgical procedures where an implant needs to be placed onto a certain anatomy and the surgeon uses a reamer to create room for placing the implant or where a certain anatomy needs to be (partially) resected. Whereas custom made surgical instruments, such as surgical guides, have provided the surgeon guidance as to the ideal position of the implant, the exact position where bone material needs to be reamed or where the resecting tool needs to be positioned, it often remains up to the surgeon to decide on the amount of material that needs to be removed or how far the resection needs to be made. Also, while the end result of the resection or reaming step may be pre-operatively decided or planned, this is often an estimate of the most ideal end result which is difficult to verify as a lot of the patient-specific features (used for the positioning of the surgical tools) are removed as a result of the reaming or resection. Accordingly, while the surgeon often has some sort or guidance during the reaming or resection steps, the actual end result of the procedure is difficult to verify and it is often considered that the result will approach the projected result.

### SUMMARY OF THE INVENTION

Described herein are guide systems for use in surgical interventions. More particularly, the guide system comprises elements allowing the positioning and fixating of a base element onto a patient's anatomy using a fitting element specific to the patient's pre-operative anatomy and second element such as a verification element for verifying the result of the surgical intervention using the base element as a reference.

In particular embodiments, the application provides a surgical guide system comprising at least: a base element comprising one or more fixation features, for fixating the base element onto an anatomical region of a patient, and one or more connecting features, for connecting to the base element one or more of the other elements of said surgical guide system; a fitting element comprising at least one contact surface, comprising one or more patient-specific anatomy engagement surfaces or anatomy contact elements corresponding to patient-specific anatomical regions, and one or more connecting features for connecting said fitting element to said base element; and a third element comprising a functional feature and one or more connecting features for connecting said element to said base element. The possibility to position the base plate in the correct position thus ensures that other elements, which can be connected thereto, can also be positioned correctly without the need for a large surgical window. In particular embodiments, the third element is a verification element configured to correspond to anatomical regions subjected to the surgical procedure, comprising one or more connecting features for connecting said verification element to said base element. In further particular embodiments, the system comprises both a verification element and an auxiliary element comprising a functional feature and one or more connecting features for connecting said auxiliary element to said base element.

Thus, more particularly, provided herein in particular embodiments is a surgical guide system for verifying the result of a surgical intervention, comprising at least: a base element comprising one or more fixation features, for fixating the base element onto an anatomical region of a patient, and one or more connecting features, for connecting to the base element one or more of the other elements of said surgical guide system; a fitting element comprising at least one contact surface, comprising one or more patient-specific anatomy engagement surfaces or anatomy contact elements corresponding to patient-specific anatomical regions, and one or more connecting features for connecting said fitting element to said base element; and a verification element configured to correspond to anatomical regions subjected to the surgical procedure, and comprising one or more connecting features for connecting said verification element to said base element. In particular embodiments, the verification element comprises at least one contact surface having one or more anatomy engagement surfaces or anatomy contact points corresponding to anatomical verification regions which can be used for verifying the outcome of the surgical procedure.

In particular embodiments the connecting features comprise a locking feature, for releasably locking said fitting element or other element, such as a verification element onto said base element. More particularly, said locking feature comprises a dedicated feature which locks said fitting element or verification element onto said base element by a snap-fit system.

In particular embodiments said connecting features allow movement of said elements with a single degree of freedom. More particularly, said movement is a translation movement of said fitting and/or verification element or other functional element relative to said base element along a single axis. In particularly, said single axis is oriented perpendicular to the contact surface of said fitting and/or verification element. In particular embodiments, the connection between said verification or other functional element and said base element can be fixed or locked in different positions along that axis.

In particular embodiments the surgical guide system as described herein provides that said fitting and/or other element, such as a verification element further comprise a guide opening for surgical instruments or pins. More particularly, said single axis is oriented parallel to said guide opening of said fitting and/or verification element.

According to particular embodiments the surgical guide system as described herein provides that said fixation features are holes for a screw, wire or pin.

According to particular embodiments the surgical guide system as described herein provides that said fitting and/or verification element further comprises inspection holes for visually inspecting the accuracy of the mating between the patients anatomy and said contact surface.

It is further envisaged as detailed above that the surgical guide system as described herein further comprises an auxiliary element, comprising one or more connecting features for connecting said auxiliary element to said base element.

More particularly, one or more of the base element, fitting element, verification element and auxiliary element comprise one or more functional features configured to guide surgical instruments. In particular, said surgical instruments are at least one of a drill, a saw and a reamer.

It is further envisaged that the surgical guide system as described herein provides that said base element, fitting element, verification element and auxiliary element are manufactured at least partially via additive manufacturing.

Also provided herein are methods for making a surgical guiding system, comprising: (a) generating a 2D or 3D pre-operative model of at least a part of the patient's anatomy; (b) designing based on said 2D or 3D pre-operative model a post-operative model corresponding to the envisioned result of the surgical intervention; (c) generating a surgical guiding system based on the information obtained in steps (a) and (b), said surgical guiding system comprising:
- a base element comprising one or more fixation features, for fixating the base element onto an anatomical region of said patient, and one or more connecting features, for connecting to the base element one or more of the other elements of said surgical guide system;
- a fitting element comprising at least one contact surface, comprising one or more patient-specific anatomy engagement surfaces or anatomy contact elements corresponding to patient-specific anatomical regions, and one or more connecting features for connecting said fitting element to said base element; and
- a further functional element such as a verification element comprising one or more connecting features for connecting said verification element to said base element. Where the functional element is a verification element it is typically configured to correspond to anatomical regions subjected to the surgical procedure such that the system can be used for verifying the outcome of part of the surgical procedure. In particular embodiments, said verification element comprises anatomy engagement surfaces or anatomy contact elements.

In these methods said patient-specific anatomy engagement surfaces or anatomy contact elements of said fitting element are based on the information obtained in step (a). Where the verification element comprises anatomy engagement surfaces or anatomy contact elements said anatomy engagement surfaces or anatomy contact points of said verification element are based on the information obtained in step (b).

The surgical system described herein may allow for a correct, stable and accurate introduction of implants into a patient's anatomy according to pre-operative planning. This may significantly improve the outcome of the surgery.

### BRIEF DESCRIPTION OF THE DRAWINGS

The following description of the figures of specific embodiments of the methods and instruments described herein is merely exemplary in nature and is not intended to limit the present teachings, their application or uses. Throughout the drawings, corresponding reference numerals indicate like or corresponding parts and features.
- **FIG. 1**: Illustration of an embodiment of (A) a fitting element mounted on a base element and (B) a verification element mounted on a base element.
- **FIG. 2**: Illustration of an embodiment of a fitting element mounted on a base element.
- **FIG. 3**: Illustration of an embodiment of a verification element mounted on a base element.
- **FIG. 4**: Illustration of the different steps of a shoulder surgery and the use of the surgical guide system according to a particular embodiment as described herein. (A) fitting element mounted on a base element positioned onto the pre-operative anatomy; (B) base element positioned onto coracoid process during the surgical intervention; (C) a verification element mounted on a base element verifying the process of the surgical intervention; and (D) base element positioned onto coracoid process after the surgical intervention.
- **FIG. 5**: Illustration of (A) the position of a base element onto a wrist and (B) a verification element mounted on a base element.

In the figures, the following numbering is used:
1 - base element; 2 - connector of the base element; 3 - locking feature; 4 - fixation feature, 5 - fixation pin; 10 - fitting element; 11 - contact surface of the fitting element; 12 - patient-specific anatomy engagement surfaces of the fitting element; 13 - connector of the fitting element; 14 - guide opening of the fitting element; 16 - bridge of the fitting element; 20 - verification element; 21 - contact surface of the verification element; 22 - anatomy engagement surfaces of the verification element; 23 - connector of the verification element; 24 - guide opening of the verification element; 25 - inspection holes of the verification element; 26 - bridge of the verification element; 30 - surgical guiding pin; 31 - glenoid cavity (pre-operative anatomy); 32 - coracoids process; 33 - glenoid cavity (after surgical intervention).

### DETAILED DESCRIPTION

While potentially serving as a guide for understanding, any reference signs in the claims shall not be construed as limiting the scope thereof.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" when referring to recited components, elements or method steps also include embodiments which "consist of" said recited components, elements or method steps.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments described herein are capable of operation in other sequences than described or illustrated herein.

The values as used herein when referring to a measurable value such as a parameter, an amount, a temporal duration, and the like, may encompass variations of +/-10% or less, preferably +/-5% or less, more preferably +/-1% or less, and still more preferably +/-0.1 % or less of and from the specified value, insofar such variations are appropriate to ensure one or more of the technical effects envisaged herein. It is to be understood that each value as used herein is itself also specifically, and preferably, disclosed.

The recitation of numerical ranges by endpoints includes all numbers and fractions subsumed within the respective ranges, as well as the recited endpoints.

All documents cited in the present specification are hereby incorporated by reference in their entirety.

Unless otherwise defined, all terms used in disclosing the concepts described herein, including technical and scientific terms, have the meaning as commonly understood by one of ordinary skill in the art. By means of further guidance, definitions for the terms used in the description are included to better appreciate the teaching of the present disclosure. The terms or definitions used herein are provided solely to aid in the understanding of the teachings provided herein.

Described herein are guide systems for use in surgical interventions. More particularly, the guide system comprises at least three elements allowing the positioning and fixating of a base element onto a patient's anatomy using a fitting element specific to the patient's pre-operative anatomy and optionally a verification element for verifying the result of the surgical intervention using the base element as a reference.

The surgical guide system described herein may provide in a multitude of functionalities including for instance the verification of a surgical procedure while maintaining a small surgical window. The system allows positioning a base element onto a patient's anatomy using a fitting element which is patient-specific in that it specifically fits on the patient's anatomy. Accordingly, the base element itself does not have to contain patient-specific features and can for instance be placed on an anatomical region where it is difficult to identify these patient-specific features. In order to ensure that a patient-specific tool fits precisely to a patient's anatomy the size of the patient-specific anatomy engagement surfaces are preferably rather large. By using a detachable element which comprises the patient-specific engagement surfaces, the size of the base plate or base element can be reduced considerably allowing it to be positioned onto certain anatomical regions which were not considered before. Furthermore, after fixating the base plate, the fitting element can be removed and the surgical intervention can continue. The base plate can in the following surgical steps be used as a reference point onto which other elements are attached and used in the surgical procedure, ensuring that the procedure continues according to the pre-operative planning.

In particular embodiments, the envisaged function of the guide includes or consists of verification of the surgical procedure. Accordingly, when the surgeon needs to perform certain surgical steps where the level of guidance is low, a verification element can be mounted on the base plate in order for the surgeon to verify and follow the progress of the surgical intervention. This can be ensured either visually or physically. In particular embodiments, the verification element is typically configured to correspond to anatomical regions subjected to the surgical procedure. For instance the verification element may comprise features which provide a visual reference for the surgeon, and more particularly allow him to identify the envisaged outcome of the surgical procedure according to the preoperative plan.

In particular embodiments, the verification element comprises a mating surface that is designed to fit onto the patient's anatomy after the surgical procedure has been correctly carried out and the surgeon can use the verification element several times to check the progress of the intervention and adapt it where necessary. This verification element does not require any patient-specific anatomy engagement surfaces matching the pre-operative anatomy, as the base element is used as reference point. This is of great importance as often in surgical interventions where part of the bone is resected or reamed, the pre-operative patient-specific features are actually removed requiring the use of patient-specific elements outside the area where the surgery occurs and consequently necessitating the enlargement of the surgical window. The latter is avoided using the surgical guide system described herein.

In further embodiments, the system comprises additionally or alternatively to the verification element, one or more (auxiliary) functional elements which connect onto the base plate in a similar way as envisaged herein for the verification element but ensure one or more other functions such as a guiding element for a surgical tool.

More particularly, provided herein is a surgical guide system which may provide several different functionalities including for instance verifying the result of a surgical intervention, comprising at least:
- a base element comprising one or more fixation features, for fixating the base element onto an anatomical region of a patient, and one or more connecting features, for connecting to the base element one or more of the other elements of said surgical guide system;
- a fitting element comprising at least one contact surface, comprising one or more patient-specific anatomy engagement surfaces or anatomy contact elements corresponding to patient-specific anatomical regions, and one or more connecting features for connecting said fitting element to said base element; and;
- a functional element comprising one or more connecting features for connecting said verification element to said base element. In particular embodiments, said functional element is a verification element for verifying the surgical procedure. In particular embodiments, the verification element is configured to correspond to anatomical regions subjected to the surgical procedure. In particular embodiments the verification element comprises features which provide a visual reference for the surgeon. More particularly the features provide a visual reference provide an indication as to the orientation of the surgical procedure. In particular embodiments such features include, but are not limited to pins and/or three-dimensional shapes. In particular embodiments, such features will allow the surgeon to verify the orientation of the surgical procedure (e.g. cutting, reaming).

In further particular embodiments, the verification element comprises at least one contact surface, comprising one or more anatomy engagement surfaces or anatomy contact elements corresponding to anatomical regions within the region of the surgical intervention, and one or more connecting features for connecting said verification element to said base element. More particularly the connecting features ensure that said base element and said fitting element or said verification element can be connected in fixed position relative to each other. In particular embodiments, the verification element thus comprises one or more anatomy engagement surfaces which will fit onto the anatomy of the patient only if the surgical procedure is performed as planned. In particular embodiments, the anatomy engagement surfaces allow for some on site determination of the optimal height.

The surgical guide system as described herein may contain several functionalities. In particular embodiments, this may be ensured by providing, on the verification element or on a separate functional element, specific functional features such as features aiding the surgical intervention. These functional features may for instance be guides for guiding surgical instruments during the surgical intervention including for instance cutting slots or cutting planes for guiding surgical tools during for instance an osteotomy, reamer guides for guiding reaming tools during the intervention or other types of guides (drill guides, pin guides, screw guides) that would be considered by a skilled person.

In particular embodiments the base element is envisaged for fixating onto an anatomical region of the patient outside the region of the surgical intervention. In further particular embodiments, the fixation features of the base element are configured to allow a stable fixation to any region of the anatomy envisaged. However, it will be understood that, depending on the envisaged surgical intervention, regions of the anatomy can be determined as suitable for placement of the base plate, and preferred structures of the fixation features for generally fitting onto the desired anatomical part can be envisaged. In further embodiments, the fixation features can effectively be locked.

In particular embodiments the surgical guide system as described herein comprises at least three elements, a base element or base plate onto which two or more other elements i.e. a fitting element and one or more functional elements can be mounted using the connecting features. In particular, the connecting features are designed such that it allows mounting and detaching the fitting and functional element separately. In particular embodiments, where both the fitting and functional element, such as the verification element comprise contact surfaces which make contact with the same area of the patient's anatomy the verification element is typically mounted onto the base element while the fitting element is detached. In a particular embodiment where the patient-specific elements of the fitting and verification element make contact with the different area of the patient's anatomy both the fitting and verification element can be mounted onto the base plate at the same time.

According to a particular embodiment the base plate comprises a single connecting feature onto which either the fitting or the other functional element(s) such as the verification element are detachably mounted. However in alternative embodiments, the base plate may connect to the fitting element and the functional element with different connecting features.

In particular, the base plate or base element refers to a small surgical tool which is fixated onto a patient's anatomy and after fixation used as a reference point. In a particular embodiment the base plate is essentially devoid of patient-specific anatomy engagement surfaces or anatomy contact elements corresponding to patient-specific anatomical regions. Particularly, the base plate is a surgical tool with a standard structure comprising one or more fixation features for fixating the base plate in the correct position and one or more connecting features onto which other elements of the surgical guide system as described herein can be mounted. In particular embodiments, the base plate is configured to be positioned onto a region outside the region that is subjected to the surgical procedure.

Once positioned correctly via the fitting element, the base plate is preferably fixed temporarily onto the bone, such that the relative position of the base plate is locked. Such fixation may be obtained via screws, wires and pins such as Kirschner wires and the like. Fixation preferably occurs outside the surgical area, to avoid interference with other devices such as reamers, impactors, implants, and the like. Accordingly, in particular embodiments, the base element is provided with one or more fixation features for fixating said base element. In certain examples, said fixation features may comprise a hole for receiving a pin, a screw, or a drill. The one or more fixation features may be a part of the body of the fixture, or may be rigidly connected thereto, for example via a spacer.

The terms "positioning" and "fixing" as used herein, when referring to positioning or fixing of a base plate onto a patient's anatomy, does not necessarily imply that the base plate itself contacts the patient's anatomy or bone part(s). Indeed, in particular embodiments, the base plate may be positioned on a bone fragment via one or more screws, without direct contact between the base plate and the bone fragment.

The fitting element is typically mounted onto the base plate and accordingly provides certain additional features to the base plate. Particularly, the fitting element is used to generate a patient-specific structure which can be used to position and fixate the base element in the correct position. Accordingly, the fitting element comprises one or more connecting features in order for it to be mounted onto the base element. Additionally, the fitting element also comprises at least one contact surface, comprising one or more patient-specific anatomy engagement surfaces or anatomy contact elements corresponding to patient-specific anatomical regions.

In particular embodiments, the contact surface may comprise one or more anatomy engagement surfaces which are free-form structures fitting at least part of the patient's anatomy. The term "free-form structure" as used herein refers to a structure having an irregular and/or asymmetrical flowing shape or contour, more particularly fitting at least part of the contour of the bone. Thus, in particular embodiments, the free-form structure is a free-form surface. A free-form surface refers to an (essentially) two-dimensional shape contained in a three-dimensional geometric space. Indeed, as will be detailed below, such a surface can be considered as essentially two-dimensional but may have a varying thickness. Typically, the free-form structure or surface is characterized by a lack of rigid radial dimensions, unlike regular surfaces such as planes, cylinders and conic surfaces. Free-form surfaces are known to the skilled person and widely used in engineering design disciplines. Typically non-uniform rational B-spline (NURBS) mathematics is used to describe the surface forms; however, there are other methods such as Gorden surfaces or Coons surfaces. The form of the free-form surfaces are characterized and defined not in terms of polynomial equations, but by their poles, degree, and number of patches (segments with spline curves). Free-form surfaces can also be defined as triangulated surfaces, where triangles are used to approximate the 3D surface. Triangulated surfaces are used in STL (Standard Triangulation Language) files that are known to a person skilled in CAD design. The free-form structures described herein are structured such that they fit the surface of the patient's anatomy specifically, thereby giving the structures their free-form characteristics.

The one or more patient-specific anatomy engagement surfaces and/or anatomy contact elements provided on the contact surface of the fitting element may allow the surgeon to ensure the correct position of the fitting element and base plate connected thereto onto the patient's anatomy according to pre-operational planning. Indeed, the one or more patient-specific anatomy engagement surfaces and/or anatomy contact elements on the fitting element may provide a unique and stable fit onto specific areas on or around the patient's anatomy (with or without cartilage or other soft tissue). Preferably, the one or more patient-specific anatomy engagement surfaces and/or anatomy contact elements fit onto the patient's anatomy in at least three contact points.

The patient-specific anatomy engagement surfaces provided on the fitting element can be selected from anatomical elements (which match a specific surface structure on the bone, with or without cartilage or other soft tissues) and/or combinations of specifically placed anatomy contact points such as pins (corresponding to a specific shape of the bone). According to particular embodiments the patient-specific anatomy engagement surfaces match with the pre-operative anatomical structure of the patient.

The functional element is typically mounted onto the base plate after the fitting element is removed and accordingly provides certain additional features to the base plate.

Particularly, the verification element is used to generate a tool which can be used to verify the progress and/or result of a surgical intervention when mounted onto the base element. In particular embodiments, the verification element comprises features which are designed such that they allow a visual check of the surgical procedure by the surgeon. In particular embodiments, the verification element provides in a structure which is highly similar to the fitting element in that it also comprises one or more patient-specific contact surfaces, with the exception that the contact surface comprises one or more anatomy engagement surfaces or anatomy contact elements corresponding to anatomical verification regions which correspond to the expected outcome of the surgical procedure (or part thereof). Accordingly, whereas the fitting element comprises patient-specific anatomy engagement surfaces matching with the pre-operative anatomical structure of the patient, the verification element is configured to correspond to anatomical regions subjected to the surgical procedure and preferably comprises anatomy engagement surfaces matching with the post-operative anatomical structure of the patient.

In a particular embodiment the surgical guide system as described herein comprises connecting features wherein said connecting features comprise a connecting feature, for releasably connecting said fitting element or other functional element, such as a verification element onto said base element. More particularly it is envisaged that the connecting features ensure that the different elements can be connected to ensure a fixed position relative to each other. This implies that after connection with said connecting features, said elements will be able to move relative to each other with only a very limited degree of freedom. In particular embodiments, connection of the fitting and/or other elements with the connection features of the base element restricts the movement of the fitting and/or verification elements in all six degrees of freedom. However, it is also envisaged that in certain embodiments, the movement of the fitting and/or verification elements is not restricted in all six degrees of freedom when connected by the connecting features of the base plate; more particularly in one, two, three, four, or five degrees of freedom.

According to a particular embodiment of the surgical guide system as described herein said connecting features provides only a single degree of freedom, preferably the translation of said fitting and/or verification element in respect to said base element along a single axis. Particularly said single axis is oriented perpendicular to the contact surface of said fitting and/or verification element. The limitation of the degrees of freedom to the translation along a single axis allows the user to mount the fitting and/or functional (e.g. verification elements) onto the base element, but restricts any other movement of the fitting and/or other elements into other directions. By providing that the said single axis is oriented perpendicular to the contact surface of said fitting and/or functional (e.g. verification) element the system ensures that the mounting is performed from a direction that provides the largest surgical window not being hindered by any other anatomical elements.

In particular embodiments said connecting features are mating features typically comprising a male and female feature which specifically interact and match with each other. A locking feature may also be attached to the connecting features on order to lock the connection between the connecting features in a predefined and fixed position (i.e. limiting movement in all degrees of freedom). The use of the locking features can result in a unique and more stable connection between the base element and the other element of the surgical guide system.

In particular embodiments as detailed above, the connecting features of the base plate combine with one or more dedicated connecting features provided on the fitting and/or verification or other functional elements, thereby forming a specific mechanism such as a dovetail coupling, interlocking features, a pinned system, a snap-fit system, or a combination thereof. In particular embodiments, the one or more connecting features may form grooves or slots, which allow interlocking e.g. with hooks or other types of extensions present on the fitting and/or verification elements. Other combinations of features which can function as connecting features will be known to the skilled person. In particular embodiments as detailed above, the base element comprises one or more locking features which allow or ensure an interlock between the base plate and said fitting and/or other elements. Such locking features may include but are not limited to hooks, pins, screws, snap-fits, clamps, clasps, jaws, pincers, plugs, cramps and cramp-irons. More particularly, said locking feature comprises a dedicated feature which locks said fitting element or verification element onto said base element by a snap-fit system.

In particular embodiments, the locking features may allow the elements to be fixed in different positions relative to each other. More particularly, the locking features may be placed along the axis of the orientation of the envisaged surgical intervention. This allows positioning of the functional tool and more particularly the verification tool at different depths, so as to allow some variation on the predetermined depth of the surgical procedure.

In particular embodiments, the surgical guide system as described herein further provides that said fitting and/or functional element such as a verification element further comprise a guide opening providing room for surgical instruments required for the surgical procedure or for pins for positioning onto the patients anatomy. The guide openings in the fitting and/or verification or other functional element are positioned according to the pre-operative plan such that, in particular embodiments, pins or wires which are used during the surgical intervention and which are already positioned onto the patient's anatomy can remain onto their position. The guide openings will shift over or along the pins or wires ensuring that the surgical procedure is not hindered or interrupted by the mounting of the fitting and/or verification element. In a particular embodiment the orientation of said single axis is parallel to said guide opening of said fitting and/or verification element, such that the mounting of the fitting and/or verification elements occurs along the pins or wires used for the surgical intervention.

In particular, the surgical guide system as described herein provides in a base element comprising fixation elements which are holes for a screw, drill, wire or pin such as a Kirschner wire. The fixation elements are needed for fixating the base element to the patient' anatomy as described above. In particular embodiments, the system as envisaged herein is provided in combination with external fixation elements, such as wires or pin which can be used to fixate the base element onto the patient's anatomy.

In a further embodiment of the surgical guide system as described herein said fitting and/or verification or other functional element further comprise inspection holes for visually inspecting the accuracy of the mating between the patient's anatomy and said contact surface. In order for a surgeon to inspect the correspondence and mating between the fitting and/or verification element and the patient's anatomy, inspection holes may be provided through which the surgeon can inspect and evaluate the fit of the fitting and/or verification element onto the patient's anatomy, thereby allowing the evaluation of the fit by the person operating the device.

Thus, as described above, in particular embodiments, the surgical guide system as described herein additionally (as an auxiliary element) or alternatively comprises a functional element other than a verification element, comprising one or more connecting features for connecting said functional element to said base element. In particular embodiments said functional element comprises one or more functional features configured to guide surgical instruments. Said functional features may for instance be guides for guiding surgical instruments during the surgical intervention including for instance cutting slots or cutting planes for guiding surgical tools during for instance an osteotomy, reamer guides for guiding reaming tools during the intervention or other types of guides (drill guides, pin guides, screw guides) that are known to the skilled person. In particular embodiments the surgical guide system as described herein provides that one or more of the base element, fitting element, verification element and (auxiliary) functional element comprise one or more functional features configured to guide surgical instruments. In particular embodiments said surgical instruments are at least one of a drill, a saw, a pin and/or a reamer.

In certain embodiments, the surgical guide system or part thereof may be manufactured via Additive Manufacturing (see further). More particularly said base element, fitting element and verification element are manufactured at least partially via additive manufacturing. Indeed, this technology easily allows the incorporation of patient-specific features into surgical guiding system which correspond to features determined based on pre-operative planning.

Further provided herein are methods for generating the surgical guiding system described herein. More particularly, provided herein is a method for generating (patient-specific) surgical guiding systems which in particular embodiments may be used for verifying the result of a surgical intervention, comprising:
(a) generating a 2D or 3D pre-operative model of at least a part of the patient's anatomy;
(b) designing based on said 2D or 3D pre-operative model a post-operative model corresponding to the envisioned result of the surgical intervention;
(c) generating a surgical guiding system based on the information obtained in steps (a) and (b), said surgical guiding system comprising:
   - a base element comprising one or more fixation features, for fixating the base element onto an anatomical region of said patient, and one or more connecting features, for connecting to the base element one or more of the other elements of said surgical guide system;
   - a fitting element comprising at least one contact surface, comprising one or more patient-specific anatomy engagement surfaces or anatomy contact elements corresponding to patient-specific anatomical regions, and one or more connecting features for connecting said fitting element to said base element; and
   - a functional element comprising one or more connecting features for connecting said functional element to said base element. In particular embodiment, said functional element is a verification element configured to correspond to anatomical regions subjected to the surgical procedure and preferably comprising at least one contact surface, comprising one or more anatomy engagement surfaces or anatomy contact elements corresponding to anatomical verification regions verifying the outcome of part of the surgical procedure, and comprising one or more connecting features for connecting said verification element to said base element;

The methods are characterized in that the patient-specific anatomy engagement surfaces or anatomy contact elements of said fitting element are based on the information obtained in step (a). In particular embodiments, the methods are further characterized in that the nature and position (orientation) of the functional features of the elements are based on the information obtained in step (a) and (b). In further embodiments, where the system comprises a verification element comprising anatomy engagement surfaces or contact elements, said anatomy engagement surfaces or anatomy contact elements of said verification element are based on the information obtained in step (b).

Particularly said envisioned result of the surgical intervention refers to the envisioned patients anatomy after a resection or reaming procedure

Thus, the methods as disclosed herein rely on one or more images of the patient's anatomy. In particular embodiments, the methods as disclosed herein include the step of taking images of the patient's anatomy. The images may be any type of image that can be used to create a 2D or 3D image. It is not required that the images are of the entire patient. Indeed, typically, only part of the patient is reflected in the image, provided that this part also includes the area of the patient's anatomy where the surgical intervention will occur.

In particular embodiments, the images are 2D or 3D images. The images can be taken using any type of imaging apparatus or imaging technique which allows imaging or scanning the defective object in an accurate manner. These may include equipment such as cameras and scanners for industrial, household or medical use. In particular embodiments the imaging techniques and appliances used are typical medical imaging tools such as, but not limited to radiography, X-ray, ultrasound or fluoroscopy for 2D images and computer tomography (CT) scans, magnetic resonance imaging (MRI) scans for 3D images. It is noted that from a combination of 2D images a 3D model can be constituted (according to US 61/579,927 which is incorporated herein by reference). A summary of medical imaging has been described in "Fundamentals of Medical imaging", by P. Suetens, Cambridge University Press, 2002.

The term "medical imaging" as used herein refers to techniques and processes used to create images of the human or animal body (or parts and function thereof), typically for clinical purposes (medical procedures seeking to reveal, diagnose or examine disease) or medical science (including the study of normal anatomy and physiology).Based on this information, the surgical guiding system can be generated. Indeed, the pre-operative plan will determine the desirable structure of the surgical guiding system. Based thereon, patient-specific surfaces can be determined in the area of the surgical intervention which can be used to position the fitting element. Also the position of the base plate will be determined based on the pre-operative images. Once the surgical planning shows the result of for instance a reaming or resection step the verification element can be generated based upon that information.

The methods disclosed herein further rely on surgical planning data which allow the design of a 2D or 3D post-operative model corresponding to the envisioned result of the surgical intervention. Such techniques are well known to the skilled person.

Optionally, the methods described herein may comprise the step of manufacturing the elements of the surgical guiding system at least partially via Additive Manufacturing (AM). AM can be defined as a group of techniques used to fabricate a tangible model of an object typically using three-dimensional (3D) computer aided design (CAD) data of the object. Currently, a multitude of Additive Manufacturing techniques is available, including stereolithography, Selective Laser Sintering, Fused Deposition Modeling, foil-based techniques, etc.

Selective laser sintering uses a high power laser or another focused heat source to sinter or weld small particles of plastic, metal, or ceramic powders into a mass representing the 3-dimensional object to be formed.

Fused deposition modeling and related techniques make use of a temporary transition from a solid material to a liquid state, usually due to heating. The material is driven through an extrusion nozzle in a controlled way and deposited in the required place as described among others in U.S. Pat. No. 5.141.680.

Foil-based techniques fix coats to one another by means of gluing or photo polymerization or other techniques and cut the object from these coats or polymerize the object. Such a technique is described in U.S. Pat. No. 5.192.539.

Typically AM techniques start from a digital representation of the 3D object to be formed. Generally, the digital representation is sliced into a series of cross-sectional layers which can be overlaid to form the object as a whole. The AM apparatus uses this data for building the object on a layer-by-layer basis. The cross-sectional data representing the layer data of the 3D object may be generated using a computer system and computer aided design and manufacturing (CAD/CAM) software.

The surgical guiding system described herein may be manufactured in different materials. Typically, only materials that are biocompatible (e.g. USP class VI compatible) with the human body are taken into account. Preferably the surgical template is formed from a heat-tolerable material allowing it to tolerate high-temperature sterilization. In the case selective laser sintering is used as an AM technique, the surgical template may be fabricated from a polyamide such as PA 2200 as supplied by EOS, Munich, Germany or any other material known by those skilled in the art may also be used.

The surgical guiding systems are thus typically made of material which is compatible with additive manufacturing and which is able to provide a sufficient stiffness to the free-form structure. Suitable materials include, but are not limited to polyurethane, acrylonitrile butadiene styrene (ABS), polycarbonate (PC), PC-ABS, polyamide, polyamide with additives such as glass or metal particles, methyl methacrylate-acrylonitrile-butadiene-styrene copolymer, resorbable materials such as polymer-ceramic composites, etc. Examples of commercially available materials are: DSM Somos® series of materials 7100, 8100, 9100, 9420, 10100, 11100, 12110, 14120 and 15100 from DSM Somos; ABSplus-P430, ABSi, ABS-ESD7, ABS-M30, ABS-M30i, PC-ABS, PC-ISO, PC, ULTEM 9085, PPSF and PPSU materials from Stratasys; Accura Plastic, DuraForm, CastForm, Laserform and VisiJet line of materials from 3-Systems; Aluminium, CobaltChrome and Stainless Steel materials; Maranging Steel; Nickel Alloy; Titanium; the PA line of materials, PrimeCast and PrimePart materials and Alumide and CarbonMide from EOS GmbH.

A further aspect provides in the use of a surgical guide system as described herein to direct and/or verify a surgical procedure, more particularly in a limited surgical window. Indeed, the system envisaged herein can provide in a multitude of functionalities such as for instance but not limited to verifying the result of a surgical intervention. More particularly said surgical intervention includes a resection or reaming procedure. The surgical guide systems as described herein are particularly useful for verifying and inspecting the result of a surgical procedure or a particular step thereof. Particularly, the result of surgical steps where part of the bone needs to be resected or reamed in order to for instance create room for positioning an implant can be inspected using surgical guide system as described herein as these surgical guide systems allow an accurate and patient-specific verification of the form, position and direction of a reamed, resected or cut anatomical feature. More particularly the methods envisaged herein relating to the verification of a surgical procedure comprise the steps of positioning a patient-specific fitting element onto the appropriate anatomical feature of the patient, such that the one or more contact surfaces of the fitting element mate with the corresponding anatomical feature, ensuring the position of the fitting element according to the preoperative planning. Said patient-specific fitting element may already be connected to the base plate or said base plate may be connected thereto in a next step. Typically, it is advantageous to position the fitting element and base plate together. As a result of the connection with the fitting element, the base plate can directly be positioned on the correct anatomical region envisaged in the preoperative planning. The base plate can then be fixated to the anatomical region in this position using fixating features. In a next step the fitting element is disconnected from the base plate. In particular embodiments the one or more contact surfaces of the fitting element are located in the region of the surgical intervention, as this is cleared by the surgical window. In further embodiments, the base plate is positioned outside the region of the surgical intervention, to avoid hindrance thereof during the surgical intervention. After removal of the fitting element, the region envisaged for the surgical intervention is again accessible to the surgeon. Thus, hereafter, the surgical intervention can take place. Optionally one or more functional elements are connected to the base plate using dedicated connection features, which element can be used for assisting in the surgical intervention. Additionally or alternatively in a next step a verification element is connected to the base plate using dedicated connecting features. Again, as a result of the connection with the base plate, the functional element can directly be positioned in the desired position envisaged in the preoperative planning. The verification features of the verification element allow the surgeon to verify the surgical procedure. As indicated above, this may be a visual verification. Additionally or alternatively, where the verification features comprise contact elements which are designed to fit onto the area of the surgical intervention corresponding to the envisaged outcome of the surgical intervention, the verification element can be positioned onto the area of the surgical intervention by the surgeon, so as to check the accuracy of the surgical intervention. In particular embodiments, this procedure (removal and reconnection of the verification tool on the base plate) can be repeated several times, such that the surgeon can follow the accuracy of the surgical intervention.

Thus in particular embodiments the application provides surgical methods whereby one or more steps in a surgical procedure is/are verified using the surgical guiding system as described herein. The surgical method described herein comprises the steps of:
(i) mounting the fitting element as described herein onto the base element as described herein;
(ii) positioning the fitting element onto a matching patient's anatomy and verifying the fit;
(iii) maintaining the fitting element mounted onto the base element in the matching position and fixating the base element onto the patient's anatomy;
(iv) detaching the fitting element from the base element;
(v) performing a surgical procedure, thereby adapting the patient's anatomy;
(vi) mounting the verification element onto the base element;
(vii) verifying or inspecting the fit of the verification element with the adapted patient's anatomy after the surgical procedure;
(viii) detaching the fitting element from the base element; and;
(ix) optionally repeating steps (v) to (viii) until the verification element matches with the adapted patient's anatomy.

The verification step (vii) indicates to the surgeon if or to what extend the surgical procedure is performed according the pre-operative plan.

Additionally, the use of the verification element can be combined with a bone model in the reamed, cut or resected state. By putting the verification element onto the bone model the surgeon is given a sense of how it should fit. The bone model can also be used for palpation.

In particular embodiments, the methods described herein are characterized in that the fitting element is positioned in a region of the anatomy which is easily accessible through the surgical window, while the base plate is positioned in a region which is not easily accessible through the surgical window. In further particular embodiments, as described above, the fitting element is positioned in a region of the anatomy which is part of the region in which the surgical intervention is to take place, while the base plate is positioned in a region of the anatomy of said patient which is outside the region where the surgical intervention is to take place.

In a further particular embodiment it is envisaged that the surgical guide system as envisaged herein is configured to ensure that the mounting of the verification element onto the base element can be locked at different levels. During pre-operative planning, the angle of a certain cut can be for instance fixed but there may remain a certain degree of freedom as to the height of the cut. This decision is left to the surgeon during the surgery. The ability to lock the verification element onto the base element at different levels allows the surgeon to decide during the surgery for instance on the height of the cut, while the verification element can be used to verify and inspect the angle of the cut. This variability allows for intra-operative decision taking, when certain information is lacking the pre-operative imaging (due to for instance bone quality). The ability to lock the verification elements at different levels also allows the surgeon to carefully progress with the surgical intervention and for instance perform a number of progressive cuts.

The instruments described herein will now be illustrated by the following, non-limiting illustrations of particular embodiments.

### EXAMPLES

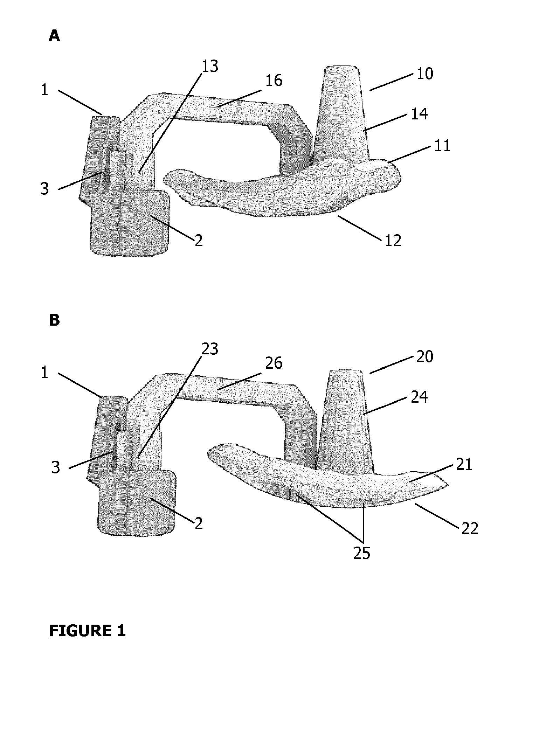

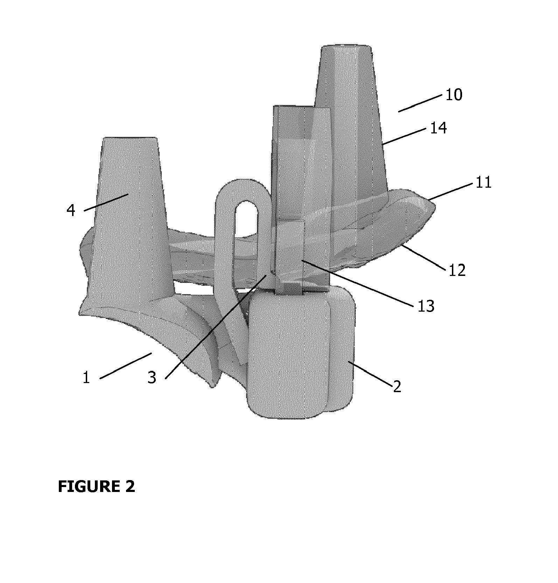

The different components of the surgical guide system as described herein are illustrated in figures 1 to 3. Figure 1A and Figure 2 illustrate a fitting element (10) mounted on a base element (1) though a connecting feature (2) on the base element and a mating connecting feature (13) on the fitting element. The base element (1) further comprises a locking feature (3) locking the connection between the base element (1) and the fitting element (10) assuring that both elements are mounted correctly and assuring that both elements are accurately and stably mounted to each other. The fitting element (10) further comprises a contact surface (11) which is used to make contact with the patient's anatomy comprising a number of patient-specific anatomy engagement surfaces (12) on the bottom surface designed to fit and match with the patient's anatomy. The fitting element (10) further comprises a guide opening (14) providing room for surgical instruments or pins which are already positioned or have to be positioned onto the patient's anatomy. A bridge element (16) connects the part of the fitting element (10) comprising the contact surface (11) with the part of the fitting element (10) comprising the connector (13).

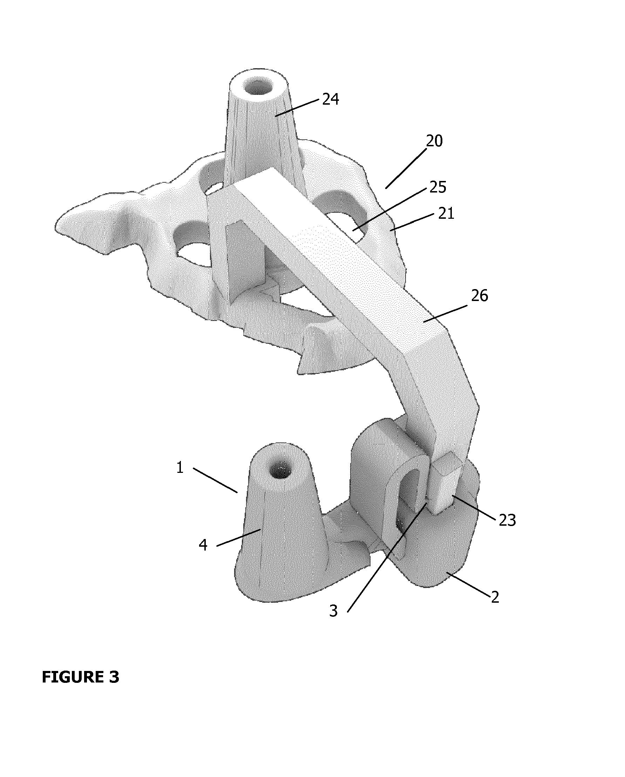

Figure 1 B and Figure 3 illustrate a verification element (20) mounted on a base element (1) though a connecting feature (2) on the base element and a mating connecting feature (23) on the verification element. The base element (1) further comprises a locking feature (3) locking the connection between the base element (1) and the verification element (20) assuring that both elements are mounted correctly and assuring that both elements are accurately and stably mounted to each other. The verification element (20) further comprises a contact surface (21) which is used to make contact with the patient's anatomy comprising a number of anatomy engagement surfaces (22) on the bottom surface designed to fit and match with the patient's anatomy after a particular step in the surgical procedure. In order to verify the progress and accuracy of the fit onto the patient's anatomy, inspection holes (25) may be provided into the contact surface (21). The verification element (20) further comprises a guide opening (24) providing room for surgical instruments or pins which are already positioned or have to be positioned onto the patient's anatomy. A bridge element (26) connects the part of the verification element (20) comprising the contact surface (21) with the part of the verification element (20) comprising the connector (23).

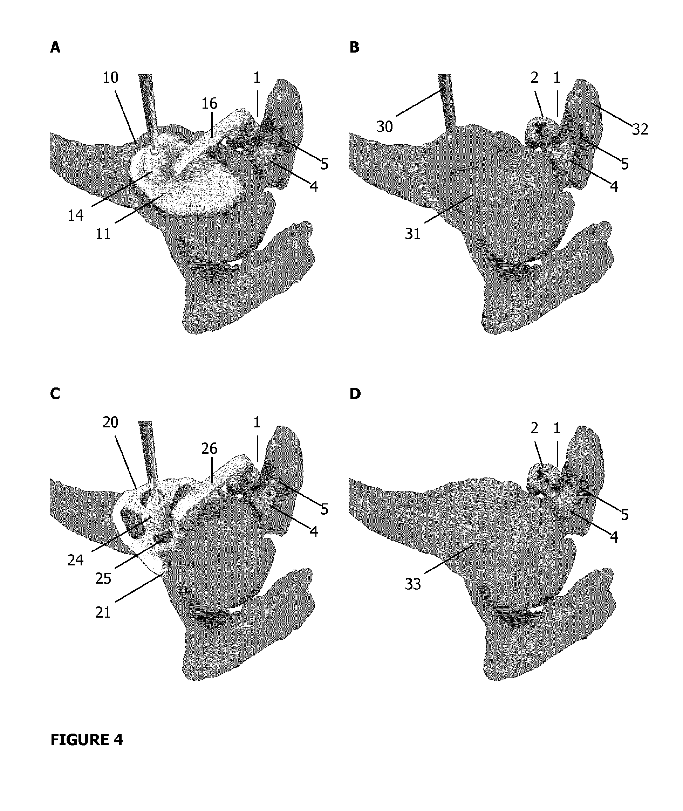

Figure 4 illustrates how the surgical guide system as described herein is used during a surgical intervention (shoulder anatomy). After exposure of the area where the surgical intervention is to occur the fitting element (10) mounted on the base element (1) is inserted into the surgical window and using the patient-specific anatomy engagement surfaces (12) on the fitting element (10) the correct position is identified by the surgeon. As illustrated in

Figure 4A the fitting element (10) is positioned onto the glenoid cavity (31) of the patient's anatomy. The glenoid cavity (31) is exposed during the surgery as this is also the area where the surgical intervention will take place. Using patient-specific features in the glenoid cavity the fitting element (10) mounted on the base element (1) is accurately positioned and the base element (1) is positioned and fixated on the coracoid process (32).

As the fitting element is the part of the structure that comprises the patient-specific engagement surfaces, the size of the base element can be reduced as this element does not necessitate the presence of patient-specific engagement surfaces. Also, the base element (1) may be positioned outside of at the edge of the surgical window. After fixation of the base element (1) the fitting element (10) is detached exposing the glenoid cavity (31) as illustrated in Figure 4B and allowing the surgeon to perform certain surgical steps including for instance reaming part of the glenoid cavity. The base plate (1) is positioned outside or at the edge of the surgical intervention and therefore it does not hinder the surgeon during the surgical intervention. In order to verify the reaming procedure, the surgeon can mount the verification element (20) onto the base element (1) as illustrated in Figure 4C in order to verify the progress of the reaming procedure. This allows where necessary the adaptation of the reaming procedure. After the reaming procedure the verification element (20) is mounted onto the base element (1) to verify the end result. The end result of the procedure is shown in Figure 4D. The surgical intervention can be continued further and at the end of the procedure the base element (1) will be removed.

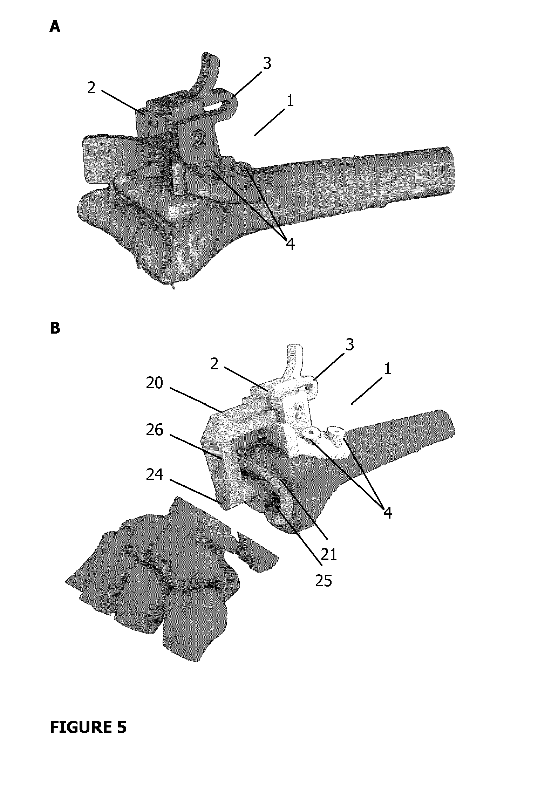

Figure 5 shows some steps of a surgical intervention similar to Figure 4 and illustrates how the surgical guide system as described herein is used during a surgical intervention (wrist anatomy).

## Claims

1. A surgical guide system, comprising at least:
- a base element comprising one or more fixation features for fixating the base element onto an anatomical region of a patient, and one or more connecting features, for connecting to the base element one or more of the other elements of said surgical guide system;
- a fitting element comprising at least one contact surface, comprising one or more patient-specific anatomy engagement surfaces or anatomy contact elements corresponding to patient-specific anatomical regions, and one or more connecting features for connecting said fitting element to said base element; and
- a verification element configured to correspond to anatomical regions subjected to the surgical procedure, and one or more connecting features for connecting said verification element to said base element;
wherein said connecting features ensure that said base element and said fitting element or said verification element can be connected in a fixed position relative to each other.

2. Surgical guide system according to claim 1, wherein said connecting features comprise a locking feature, for releasably locking said fitting element or verification element onto said base element.

3. Surgical guide system according to claim 2, wherein said locking feature comprises a dedicated feature which locks said fitting element or verification element onto said base element by a snap-fit system.

4. Surgical guide system according to any of claims 1 to 3, wherein said connecting features allow movement of said elements with a single degree of freedom.

5. Surgical guide system according to claim 4, wherein said movement is a translation movement of said fitting and/or verification element relative to said base element along a single axis.

6. Surgical guide system according to claim 5, wherein said single axis is oriented perpendicular to the contact surface of said fitting and/or verification element.

7. Surgical guide system according to any of claims 1 to 6, wherein said fitting and/or verification element further comprise a guide opening for surgical instruments or pins.

8. Surgical guide system according to claim 8, wherein said single axis is oriented parallel to said guide opening of said fitting and/or verification element.

9. Surgical guide system according to any of claims 1 to 8, wherein said fixation features are holes for a screw, wire or pin.

10. Surgical guide system according to any of claims 1 to 9, wherein said fitting and/or verification element further comprises inspection holes for visually inspecting the accuracy of the mating between the patients anatomy and said contact surface.

11. Surgical guide system according to any of the claims 1 to 10, further comprising an auxiliary element, comprising one or more connecting features for connecting said auxiliary element to said base element.

12. Surgical guide system according to any of the claims 1 to 11, wherein one or more of the base element, fitting element, verification element and auxiliary element comprise one or more functional features configured to guide surgical instruments.

13. Surgical guide system according to claim 12, wherein said surgical instruments are at least one of a drill, a saw and a reamer.

14. Surgical guide system according to any one of claims 1 to 13, wherein said base element, fitting element and verification element and auxiliary element are manufactured at least partially via additive manufacturing.

15. A method for making a surgical guiding system for verifying the result of a surgical intervention, comprising:
(a) generating a 2D or 3D pre-operative model of at least a part of the patient's anatomy;
(b) designing based on said 2D or 3D pre-operative model a post-operative model corresponding to the envisioned result of the surgical intervention;
(c) generating a surgical guiding system based on the information obtained in steps (a) and (b), said surgical guiding system comprising:
- a base element comprising one or more fixation features, for fixating the base element onto an anatomical region of said patient, and one or more connecting features, for connecting to the base element one or more of the other elements of said surgical guide system;
- a fitting element comprising at least one contact surface, comprising one or more patient-specific anatomy engagement surfaces or anatomy contact elements corresponding to patient-specific anatomical regions, and one or more connecting features for connecting said fitting element to said base element; and
- a verification element configured to correspond to anatomical regions subjected to the surgical procedure for verifying the outcome of part of the surgical procedure, and one or more connecting features for connecting said verification element to said base element;
wherein said patient-specific anatomy engagement surfaces or anatomy contact elements of said fitting element are based on the information obtained in step (a) and wherein said anatomy engagement surfaces or anatomy contact elements of said verification element are based on the information obtained in step (b).
